# EUROPEAN PATENT APPLICATION

(11) **EP 4 792 761 A1**
(43) Date of publication of application: **19.08.2026**
(21) Application number: 25701735.0
(22) Date of filing: 03.01.2025
(51) Int. Cl.: A61B 17/90

(54) **METHOD AND APPARATUS FOR GUIDING CENTER POINT IN PATIENT'S GLENOID**

(30) Priority: 24.12.2024 KR 20240195227
(71) Applicant: Seeann Solution Co., Ltd., Yeonsu-gu Incheon 21984 (KR)
(72) Inventor: SEO, Anna, Incheon 21986 (KR); NOH, Hakjong, Incheon 21986 (KR); JEONG, Youngjin, Incheon 21664 (KR)
(74) Representative: HGF
(86) International application number: PCT/KR2025/000086
(87) International publication number: WO 2026/141763

(57) **Abstract**

Provided is a method of determining a center point in a patient's glenoid, performed by a processor of an electronic apparatus, including acquiring an image of the patient's glenoid, obtaining a circumference of the glenoid, defining a plurality of areas based on criteria set with respect to respective points of the circumference, obtaining intersections formed by overlapping the plurality of defined areas and calculating a probability of each intersection, and determining a center point based on a calculated probability value.

## Description

### Technical Field

The disclosed technology relates to a method and an apparatus for guiding an implant to be inserted into a patient's scapula so that the implant can be implanted at a desired location, and more specifically, seeks to set and optimize a center point of a glenoid for implanting the implant at the desired location.

### Background Art

An artificial joint is a medical device which restores the original function of a joint by removing the joint and inserting an artificial prosthesis (implant) into an articular surface and surrounding area when joint cartilage is damaged, deformed, or fractured due to degenerative arthritis, geriatric diseases, autoimmune diseases, and trauma.

Artificial arthroplasty, which is a process of inserting the artificial joint into the human body, can restore the normal function of the joint in an uncomfortable area. Typically, shoulder arthroplasty may be performed when a shoulder joint is damaged and no longer responds to conservative treatment and the pain is severe.

However, since such a surgery is highly risky and even a minor error may cause irreversible damage to the patient, it is important to accurately replace the joint with a customized artificial joint for each patient. To this end, surgical guidance is also introduced to improve the accuracy of artificial joint implantation, designed based on each individual's different anatomical structure. Since this surgical guidance is used for artificial joint replacement surgical implants, it needs to be designed in advance in three-dimensional space. Therefore, today, the technology of establishing a surgical plan based on the patient's medical image (CT) and producing surgical guidance using 3D printing is developing day by day.

In addition, correct insertion and alignment of the implant during the artificial joint replacement surgery procedure has an important impact on the outcome of the surgery. In conventional techniques, image guidance was used to insert an implant into the glenoid, or a center point was set and the implant was positioned based on a surgeon's experience. However, this method may lack accuracy and consistency, and incorrect centering may lead to poor implant positioning, wear, and joint instability.

Therefore, a technology is required to effectively set the center point of the glenoid and accurately insert the implant based thereon. The present invention seeks to provide a new method and an apparatus for optimizing the center point of the glenoid to meet these needs and for stably and efficiently inserting an implant into the scapula based on the center point.

### Disclosure of the Invention

### Technical Goals

An object of the present disclosure is to overcome the problems of low accuracy and difficulty in ensuring consistency in the setting of the glenoid center point, which is dependent on the surgeon's experience and visual evaluation.

Further, another object is to set a glenoid center point which may reproduce natural versions and inclinations.

In addition, another object is to reduce the possibility of problems such as implant misalignment, joint wear, or functional decline which may occur due to failure to sufficiently reflect the anatomical structure of the glenoid and individual differences of patients.

Therefore, the present invention seeks to provide a method and an apparatus capable of accurately detecting a glenoid center point and effectively inserting an implant by setting the optimal version and inclination to resolve these limitations.

Technical objects to be achieved by the present disclosure are not limited to the technical objects described above, and other technical objects may be inferred from the following embodiments.

### Technical Solutions

According to an embodiment, a method of determining a center point in a patient's glenoid, performed by a processor of an electronic apparatus, may be provided, and the method includes: acquiring an image of the patient's glenoid; obtaining a circumference of the glenoid; defining a plurality of areas based on criteria set with respect to respective points of the circumference; obtaining intersections formed by overlapping the plurality of defined areas and calculating a probability of each intersection; and determining a center point based on a calculated probability value.

In addition, the determining of the center point based on the calculated probability value may include selecting the intersection at which the probability value is maximum as the center point.

In addition, the determining of the center point based on the calculated probability value may include, if the intersection point with the maximum probability value exceeds preset threshold values of inclination and version, selecting the intersection with the next probability rank as the center point.

In addition, the obtaining of the circumference of the glenoid may include extracting an edge of a glenoid surface by segmenting the image of the glenoid and analyzing an inclination change or density difference of the glenoid surface, and obtaining the circumference of the glenoid along an extracted edge line of the glenoid surface.

In addition, the defining of the plurality of areas based on the criteria set with respect to the respective points of the circumference may include representing an area formed by a plurality of circles having a same radius centered at the respective points of the circumference.

In addition, the defining of the plurality of areas based on the criteria set with respect to the respective points of the circumference may include representing an area formed with a variable radius according to anatomical characteristics of the glenoid centered on the respective points of the circumference.

In addition, the processor may visually display the probability of each intersection by the processor, and the probability may be displayed using a color, shading, or a numerical value.

In addition, the processor may display to a user inclination and version values of each intersection along with the probability value to allow the user to select a center point candidate.

According to another embodiment of the present invention, an electronic apparatus for determining a center point in a patient's glenoid may include: an input/output interface which receives an image of the glenoid; a processor configured to obtain a circumference of the glenoid from the image of the glenoid, define areas based on criteria set with respect to respective points on the circumference, obtain intersections of the defined areas, and determine a center point based on probability values of the obtained intersections; and a memory configured to store or output data of the center point.

In addition, the processor may be configured to determine a final center point based on respective probability values of a plurality of intersections, and inclination values and version values at the plurality of intersections.

Specific details of other embodiments are included in the detailed description and drawings.

### Advantageous Effects

According to the disclosed methods, it is possible to accurately determine the center point of the implant by precisely reflecting the anatomical structure of the glenoid.

In addition, it is possible to improve consistency and reproducibility of surgical results based on statistical probability in the process of deriving the center point.

In addition, by considering the version and inclination in the process of deriving the center point, it is possible to sufficiently reflect the anatomical structure of the glenoid and individual differences of the patients.

In addition, it is possible to increase clinical efficiency by reducing surgical preparation time and procedure time by automated data analysis and center point derivation.

In addition, by improving the alignment accuracy of the implant, it is possible to minimize errors which may occur during surgery and optimize the patient's recovery outcome.

Effects of the present invention are not limited to the effects mentioned above, and other effects not mentioned will be apparent to those skilled in the art from the description of the appended claims.

### Brief Description of Drawings

FIG. 1 illustrates a block diagram of an electronic apparatus which sets a center point of a glenoid according to an embodiment.
FIG. 2 is an example of an anatomical structure diagram illustrating a patient's scapula and glenoid.
FIG. 3 is an exemplary diagram illustrating a circumference of a glenoid according to an embodiment.
FIG. 4 is an exemplary diagram illustrating a state in which circles of the same radius are generated centered on respective points on a circumference of a glenoid according to an embodiment.
FIG. 5 is a diagram visually representing probabilities at intersections of the generated circles of FIG. 4 according to an embodiment.
FIG. 6 is a flowchart illustrating a method of setting a glenoid center point according to an embodiment.

### Best Mode for Carrying Out the Invention

Terms used in the embodiments are selected from commonly used terms as much as possible while considering the functions of the present disclosure, but these may vary depending on the intention of a technician working in the field, precedents, the emergence of new technologies, or the like. Additionally, in certain cases, there are terms arbitrarily selected by the applicant, and in such cases, their meanings will be described in detail in relevant descriptions. Therefore, the terms used in the present disclosure should be defined based on the meaning of the terms and the overall contents of the present disclosure, rather than simply the names of the terms.

Throughout the specification, when it is mentioned that a part "includes" or "comprises" a component, this does not mean that the part excludes other components, but rather that the part may include other components, unless otherwise stated. In addition, a term such as "... unit", "... module", or the like, described in the specification means a unit which processes at least one function or operation, which may be implemented by hardware or software or a combination of hardware and software, and may not be clearly distinguished in specific operations, unlike the illustrated example.

The expression "at least one of a, b, and c" throughout the specification may include "a alone", "b alone", "c alone", "a and b", "a and c", "b and c", or "a, b, and c".

In the following description, the terms "transmission", "communication", "sending", and "receiving" of a signal, a message, or information, or other terms of similar meanings include not only the direct transmission of the signal, message or information from one component to another, but also transmission via another component.

In particular, "transmitting" or "sending" a signal, a message, or information to a component indicates a final destination of the signal, message or information and does not imply a direct destination. The same applies to "receiving" a signal, a message, or information. Further, in the present disclosure, when it is mentioned that two or more pieces of data or information are "related", it means that, if one piece of data (or information) is acquired, at least a portion of the other piece of data (or information) can be acquired based on that piece of data (or information).

In addition, terms such as first and second may be used to describe various components, but the components should not be limited by the above terms. The above terms may be used for the purpose of distinguishing one component from another.

For example, without departing from the scope of the present disclosure, a first component may be named a second component, and similarly, the second component may be named the first component.

Referring to the accompanying drawings, embodiments of the present disclosure will be described in detail so that a person skilled in the art to which the present disclosure pertains can easily practice them. However, the present disclosure may be implemented in a number of different forms and is not limited to the embodiments described here.

In the following, the embodiments of the present disclosure are described in detail with reference to the drawings.

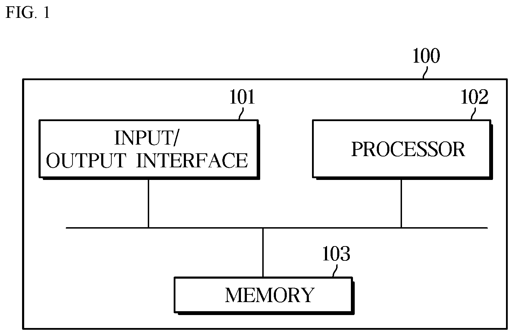

FIG. 1 illustrates a block diagram of an electronic apparatus 100 performing the present invention and schematically shows a hardware and software configuration used to implement a method of the present invention.

The electronic apparatus 100 includes an input/output interface 101, a processor 102, and a memory 103.

The input/output interface 101 is responsible for interaction with a user and input and output of data, and is configured to, for example, receive CT or MRI image data and output resulting data to a display device. An input part may include an input means such as a keyboard, a mouse, or a touchscreen, and an output part may include a monitor or another visual output device.

The processor 102 is a component which performs main operations and control of the electronic apparatus, and plays a role in processing data and deriving a center point according to the method of the present invention. The processor may perform complex operations such as analyzing anatomical data of a glenoid, generating circles, detecting intersections, and calculating probabilities, which allows it to calculate a precise center point.

Specifically, the processor 102 may include one or more processors. Here, one or more processors may be a general-purpose processor such as a CPU, AP, or DSP (Digital Signal Processor), a graphics-only processor such as a GPU or VPU (Vision Processing Unit), or an artificial intelligence (AI)-only processor such as an NPU.

One or more processors control processing of input data, according to predefined operation rules or AI models stored in a memory. Alternatively, if one or more processors are AI-only processors, the AI-only processors may be designed with hardware structures specialized for processing of specific AI models.

Predefined operation rules or artificial intelligence models are characterized by being created through learning. Here, being created through learning means that a basic artificial intelligence model is trained using a learning algorithm using a large amount of training data, thereby creating a predefined operation rule or artificial intelligence model set to perform a desired characteristic (or purpose).

Such training may be performed by a device itself on which the artificial intelligence according to the present disclosure is operated, or may be performed through a separate server and/or system. Examples of learning algorithms include supervised learning, unsupervised learning, semi-supervised learning, or reinforcement learning, and one embodiment of the present invention may include an optimal generation method of a 3D implant for compound fractures according to a reinforcement learning algorithm.

An AI model may include a plurality of neural network layers. Each of the plurality of neural network layers has a plurality of weight values, and performs neural network operations through operation results of the previous layer and operations between the plurality of weight values. The plurality of weight values of the plurality of neural network layers may be optimized by training results of the AI model. For example, the plurality of weight values may be updated to reduce or minimize a loss or cost value obtained by the AI model during the training process. The artificial neural network may include a deep neural network (DNN), such as a convolutional neural network (CNN), a deep neural network (DNN), a recurrent neural network (RNN), a restricted Boltzmann machine (RBM), a deep belief network (DBN), a bidirectional recurrent deep neural network (BRDNN), or deep Q-networks, but is not limited to the examples described above.

The memory 103 serves to store data required for an operation process performed by the processor 102 and supports execution of software programs and algorithms. The memory may include a main memory (RAM) and a secondary memory (e.g., SSD, HDD), and may store a patient's anatomical image data, centroid derivation results, and relevant intermediate data.

The electronic apparatus 100 of the present invention may be connected via a network to a remote database or server, through which the patient's CT or MRI data may be retrieved or processed results may be forwarded to another system. In addition, a user interface may provide visual information for setting surgical plans or guidelines, and output results.

The configuration shown in FIG. 1 does not limit a specific hardware configuration of the present invention, and the electronic apparatus 100 may be implemented as a device of various types (e.g., a personal computer, a tablet, a mobile device, or a professional surgical support device in a hospital). In addition, the functions of the processor 102 and the memory 103 may be performed within a single system or distributed as needed.

The electronic apparatus 100 shown in FIG. 1 may derive a center point of a glenoid implant to improve the accuracy and efficiency of a surgical procedure.

Although not shown in FIG. 1, a communication unit may include one or more components enabling communication between external devices or internal modules, including, for example, at least one of a short-range communication module, a wired communication module, and a wireless communication module.

The short-range communication module may include a variety of short-range communication modules which transmit and receive signals using wireless communication networks over a short distance, such as a Bluetooth module, an infrared communication module, a radio frequency identification (RFID) communication module, a wireless local access network (WLAN) communication module, an NFC communication module, and a Zigbee communication module.

The wired communication module may include various wired communication modules such as a local area network (LAN) module, a wide area network (WAN) module, or a value added network (VAN) module, as well as various cable communication modules such as Universal Serial Bus (USB), High Definition Multimedia Interface (HDMI), Digital Visual Interface (DVI), recommended standard 232 (RS-232), power line communication, or plain old telephone service (POTS).

The wireless communication module may include, in addition to a WiFi module and a Wireless broadband module, a wireless communication module which supports various wireless communication methods such as GSM (global system for mobile communication), CDMA (Code Division Multiple Access), WCDMA (Wideband Code Division Multiple Access), UMTS (universal mobile telecommunications system), TDMA (Time Division Multiple Access), and LTE (Long Term Evolution).

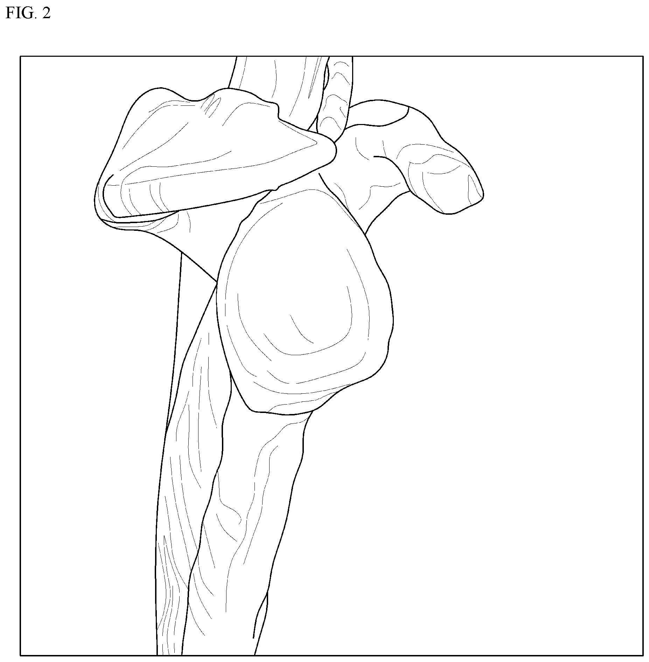

In FIG. 1, the hardware configuration of the electronic apparatus which determines the center point of the glenoid has been discussed. Next, FIGS. 2 to 5 visually illustrate an entire process of deriving the center point of the glenoid, and show steps of identifying an anatomical structure of the glenoid, defining a circumference, generating circles, analyzing intersections, and calculating probabilities.

FIGS. 2 to 5 visually illustrate the entire process of deriving the center point based on glenoid data, showing step-by-step the steps of identifying the anatomical structure of the glenoid, defining the circumference, generating the circles, analyzing the intersections, and calculating the probabilities.

First, FIG. 2 is an anatomical structural diagram illustrating the patient's scapula and glenoid, showing the position and shape of the glenoid. FIG. 2 is a three-dimensional (3D) image or planar projection of the scapula obtained through CT or MRI data, through which an anatomical region where the glenoid is located can be identified.

A reference plane is an important factor which determines the accuracy of glenoid circumference extraction, and the processor 102 may set it according to anatomical characteristics of the glenoid and surgical requirements.

Specifically, the processor 102 may determine by considering an anatomical inclination of the glenoid, and since the Friedman line reflects the alignment and anatomical position of the glenoid, the plane of the glenoid may be set by using it as a reference point for circumference extraction.

Additionally, the processor 102 segments the 3D image around the glenoid to extract only surface data of the glenoid, which may be performed through an automated image analysis algorithm or manual adjustment.

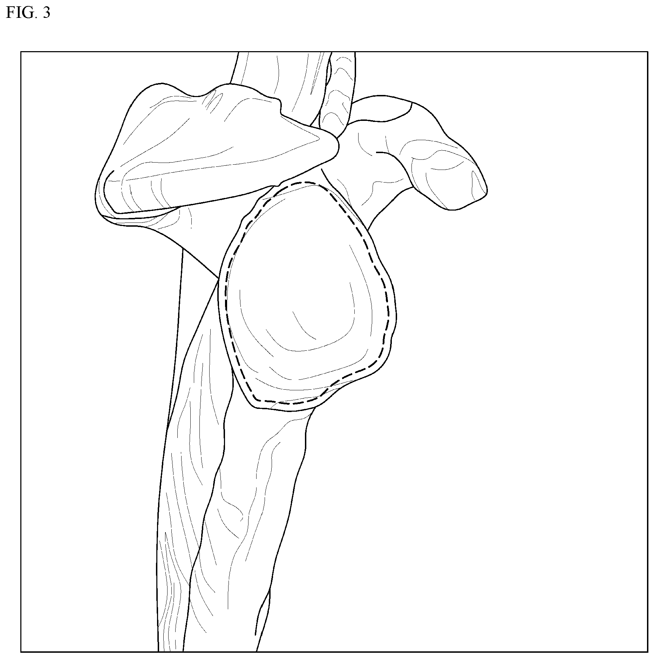

A plane projection step may be executed to extract the circumference of FIG. 3.

For example, the processor 102 may define the circumference of the glenoid on a two-dimensional plane by projecting the extracted glenoid surface data onto the set reference plane.

Accordingly, the processor 102 detects an edge of the glenoid surface to form the circumference, and the edge may be determined based on an anatomical dividing line of the glenoid and the surrounding bone structure. To do this, the processor 102 may set the edge through an automatic edge detection algorithm which automatically detects an edge by analyzing gradient changes or density differences based on CT/MRI data, or the processor 102 may also increase accuracy by having an expert adjust an edge line from the user.

Specifically, FIG. 3 is a diagram illustrating the circumference of the glenoid, showing the circumference created through a number of points defined along the anatomical edges.

The circumference of the glenoid may be automatically extracted from anatomical boundary data or defined manually. A dotted line in FIG. 3 represents an actual surface shape of the glenoid, and each point provides initial data for deriving the center point. Each point defines a specific coordinate of the circumference of the glenoid, and the points are then used as a defined area (e.g., the center of a circle) in the process of calculating the center point.

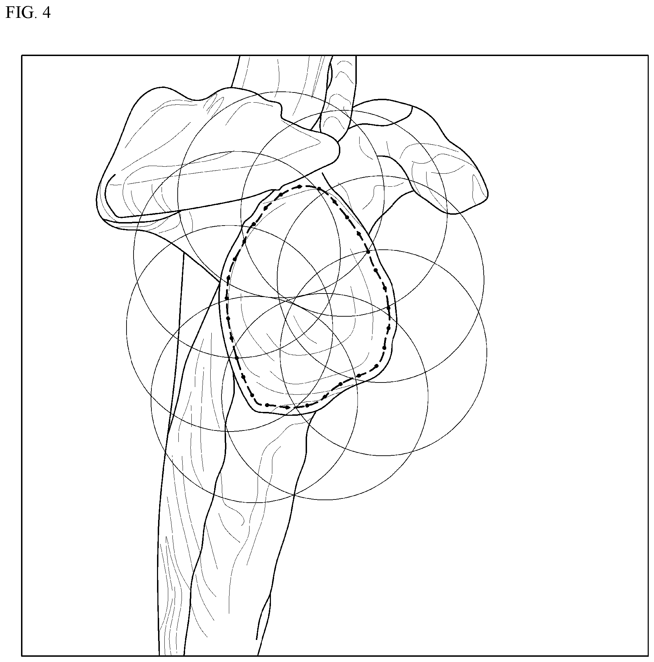

FIG. 4 is a diagram illustrating a state in which circles of the same radius are generated centered on respective points on the circumference of the glenoid. Each point located on the circumference is used as a reference point to generate the circle to derive a center point candidate. The generated circles have a selected radius, which may be adjusted according to the size and anatomical characteristics of the glenoid and implant design requirements.

Therefore, areas where respective circles overlap are important data for deriving the center point candidate, and the more intersections are formed at a location, the higher the probability that the center point exists at that location.

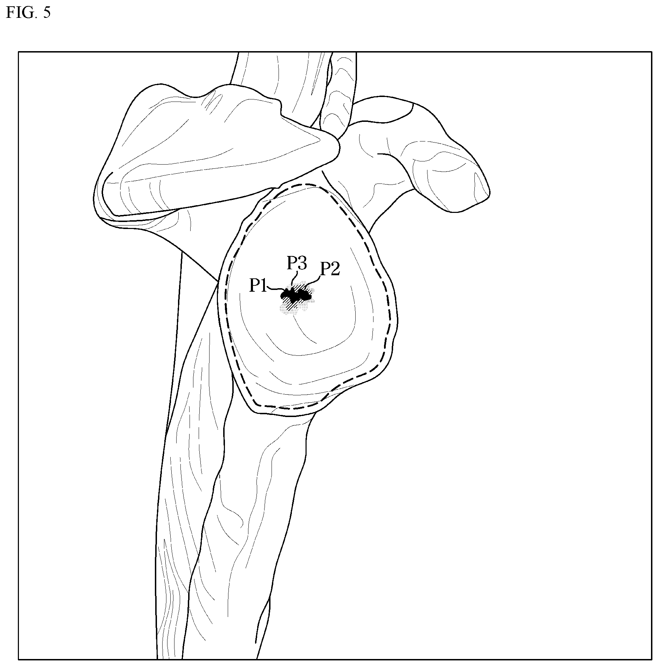

FIG. 4 specifically visually illustrates forming the intersections with each of the points forming the circumference as the center of the circle. The intersection is a point where the probability increases as more circles overlap, and it becomes a key candidate point for calculating the center point in the subsequent steps. Afterwards, FIG. 5 performs an operation which may be used as a basis for determining the center point through a probabilistic algorithm.

However, in FIG. 4, each circle is defined as one shape of a defined area, but according to one embodiment, the processor 102 may also define the area as a sphere rather than the circle.

In other words, although not provided as a separate diagram, as an example, the processor 102 may adopt a method of finding intersections by generating spheres having the same radius centered on each point on the glenoid surface. This is a method which takes into account three-dimensional structural characteristics which make it difficult to see each point of the glenoid surface as being on exactly the same plane.

Therefore, each sphere maintains a distance from the center point and forms an intersection through overlap with adjacent spheres.

In other words, after obtaining the circumference of the glenoid, the coordinates of each point forming the circumference are defined in three-dimensional space. The spheres are generated with the same radius centered around respective points. This radius may be set according to the size of the glenoid, the anatomical characteristics of the glenoid, and the surgical design requirements. The intersection is calculated in the area where the generated spheres overlap each other, and the probability of the intersection may be obtained based on the number of overlapping spheres at that point.

In addition, this method may flexibly respond to various surgical requirements and patient-tailored plans by setting the radius of the sphere variably or assigning weights according to the anatomical importance of a specific area.

FIG. 5 is a diagram visually illustrating probability distribution for the intersections of the generated circles, and shows a process of analyzing and visualizing the probabilities for the intersections. Each intersection is a point where the circles generated around a point on the circumference overlap each other, and the more intersections formed, the higher the probability that a center point exists. Specifically, in FIG. 5, the darker the color, the higher the probability of intersection.

The probability of intersection is obtained based on the number of overlapping circles, and a probability value may be determined through a frequency-based statistical approach. For example, the more circles that overlap a particular intersection, the higher the probability calculated for that point. These probabilities are quantified through mathematical algorithms or statistical models, which can be used to evaluate the reliability of center point candidates.

Additionally, the distribution of intersections may be represented using a statistical model such as the Gaussian distribution. By smoothly adjusting the surrounding probabilities around the center point, outliers may be removed and the area with the highest probability may be emphasized. In addition, a method of analyzing the density of the surrounding intersections to derive a center point of a high probability area may also be applied.

The processor 102 uses changes in color or shade to visually represent the probability distribution and displays it so that the user can check it through the input/output interface 101. The processor 102 may display intersections with a higher probability in a darker color or in an emphasized form, thereby allowing the user to intuitively identify the location of the center point candidate. These probability distribution may be represented through a visualization algorithm such as heatmaps, and may be used as a basis for setting an area where probability is concentrated as the center point.

The processor 102 may also assign weights to specific areas based on their anatomical significance.

For example, in FIG. 5, the center point candidate with a high probability is derived through analysis of the intersections of circles, but the processor 102 may determine a final center point by simultaneously considering an inclination and version of the glenoid as additional weighting factors.

Here, the inclination refers to an angle of inclination of the glenoid with respect to a vertical axis (an anatomical axis of the scapula). This refers to how much an articular surface of the glenoid which receives the humeral head is tilted upward or downward, and the normal range of inclination may vary depending on the anatomy of the patient. If the inclination angle is abnormally high or low, functional alignment of the implant may be difficult, so it should be used as an important evaluation criterion in the process of deriving the center point to improve the alignment accuracy of the implant, thereby minimizing errors which may occur during surgery and optimizing the patient's recovery outcome.

Additionally, the version refers to the degree to which a surface of the implant is rotated anteriorly or posteriorly, and is a value representing a rotation angle of the articular surface. The version value is defined as an angle between the anatomical axis of the glenoid and an alignment axis of the surgical plan, and may be divided into anteversion or retroversion. Deviation from the normal version angle may result in implant instability or functional inadequacy, which is an important consideration when selecting the center point.

In this case, it is common practice to set up the Friedman line to derive the inclination and version. The Friedman line is defined as a line connecting the spine of the scapula and the center of the glenoid, based on the anatomical alignment of the scapula and glenoid.

An endpoint of the spine of the scapula is selected from a lateral edge of the spine of the scapula at an upper or posterior aspect of the scapula, and for this, an automated algorithm may be used, and it may also be manually specified by a user (or physician) based on anatomical data of the scapula and CT/MRI images.

First, in an automated manner, the endpoint of the spine of the scapula may be automatically detected by analyzing the entire structure of the scapula based on CT or MRI data. The processor analyzes geometric data of the scapula surface to determine the lateral endpoint of the spine of the scapula based on density differences, inclination changes, or center of curvature. This process is performed using a quantitative analysis algorithm to derive the best fit point based on the geometrical central axis of the scapula and glenoid.

The user may also specify it manually. In this case, a three-dimensional image of the scapula is provided visually from CT or MRI data, and the user interface allows the user to directly select the endpoint of the spine of the scapula. The user may specify the lateral end point of the spine of the scapula while checking the anatomical structure of the scapula, and the specified point is stored by the processor as a reference point for generating the Friedman line.

After selecting the location of the end point of the spine of the scapula, the Friedman line is created by connecting this point and the center point of the glenoid. The Friedman line reflects the anatomical alignment of the scapula, from which the inclination and version of the glenoid may be derived.

For example, even if the processor 102 derives P1, which is a center point candidate with the highest probability value, in the case that the inclination angle or version value of the point exceeds a preset threshold value (e.g., inclination angle 10° to 15°, version angle -5° to 5°), the point may be determined to be unsuitable as the center point. In this case, the points P2 and P3 with the next highest probabilities are evaluated to check whether the inclination and version value are within a set criteria, and then selected as the final center point.

Additionally, when there are multiple center point candidates with the same probability value, the processor 102 may select a point with smaller version and inclination values as the final center point.

In this way, the present invention may determine the optimal center point by simultaneously considering anatomical and functional weights such as the inclination and version as well as probability-based intersection analysis. This complements the limitations of the center point derived simply by probability and contributes to optimizing the accuracy and alignment of implant installation.

As a result, FIG. 5 visually represents the center point candidates based on the probability distribution and shows the process of selecting the optimal center point by combining the additional evaluation factors, the inclination and version. This increases the stability and accuracy of surgical outcomes and enables customized implant installation tailored to the patient's anatomical characteristics.

Additionally, although not shown, the processor 102 may also apply a method of correcting the probability by utilizing existing clinical data. Based on past surgical cases or anatomical study data, the probability of the center point at a specific location may be predicted and combined to adjust the final probability distribution. This method contributes to further increasing the reliability of the intersection analysis results.

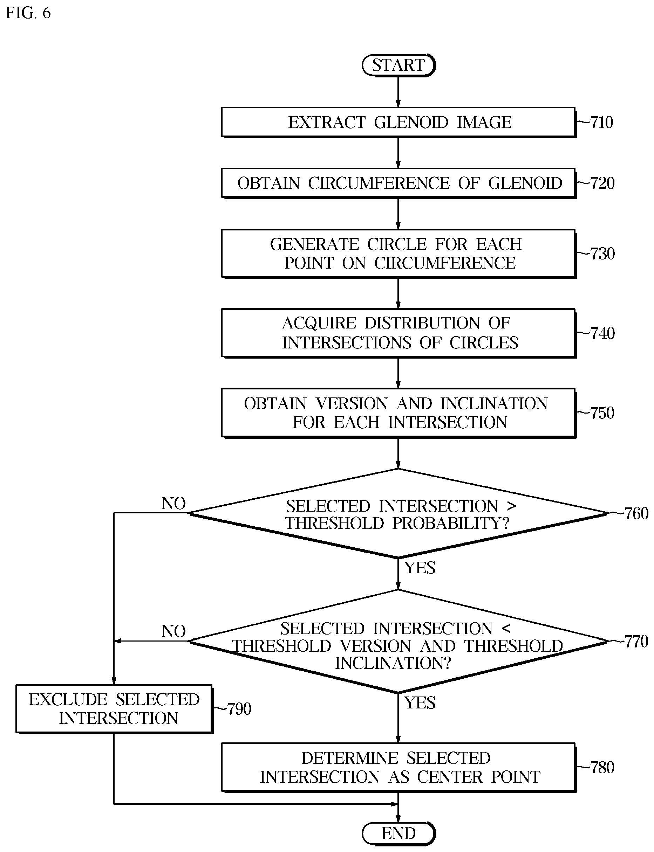

The processor 102 may perform the method of a flowchart of FIG. 6.

FIG. 6 is a flowchart showing a method of the present invention, explaining the process of deriving the glenoid center point based on the patient's CT or MRI data step by step.

First, in step 710, the processor 102 extracts a glenoid image of the patient. At this stage, the anatomy of the scapula and glenoid may be identified using the CT or MRI data, and an image of the glenoid may be obtained for analysis.

Next, in step 720, the processor 102 obtains a circumference of the glenoid. The circumference is created based on the anatomical edges of the glenoid, which accurately reflects the shape and edges of the articular surface. The circumference generated at this step is used as basis data for calculating a center point thereafter.

Next, in step 730, the processor 102 generates circles having a specified radius centered on respective points on the circumference. The radius of the circles is set according to the anatomical characteristics of the glenoid or implant design requirements, and the respective circles are generated for the respective points on the circumference.

Then, in step 740, the processor 102 obtains distribution of intersections of the generated circles. The intersection is formed in an area where the circles overlap, and this intersection is considered as a candidate for the center point. Here, a probability value is calculated for each intersection, and the probability value may be obtained based on the number of circles overlapping the corresponding point, but is not limited thereto.

Then, in step 750, the processor 102 obtains the version and the inclination angle for each intersection. This provides data to evaluate whether a specific intersection meets anatomical and functional criteria.

In step 760, the processor 102 evaluates whether the probability value of the intersection exceeds a set threshold probability. If the selected intersection does not satisfy the threshold probability, the intersection is excluded from the candidates for the final center point.

If the selected intersection satisfies the threshold probability, in step 770, the processor 102 additionally evaluates whether the version and inclination angle of the intersection satisfies a preset threshold version and threshold inclination. In this step, if the version or inclination value exceeds the threshold, the corresponding intersection may be excluded in step 790.

Finally, if the selected intersection satisfies both the threshold probability and the threshold version and inclination conditions, the processor 102 determines the intersection as the final center point in step 780. This center point is used as a reference point for inserting an alignment pin and setting a position of the implant.

The flowchart in FIG. 6 illustrates the process of deriving the optimal center point by combining probability analysis and anatomical criteria (version and inclination) step by step, which may maximize the accuracy and reliability of the center point. A series of processes from step 710 to step 790 may be performed by an automated algorithm, contributing to increasing the precision and consistency of surgical planning and implant installation.

After the center point is derived, the processor 102 may utilize the derived center point to generate surgical guidance. For example, a path of a virtual alignment pin or surgical instrument may be simulated based on the center point, and an accurate pin insertion path may be designed based thereon. This guidance data may be visualized through 3D simulation software and output to a display device for real-time use during surgery. This provides a surgeon with intuitive and practical guidance about the center point.

Additionally, the processor 102 may perform a task of optimizing the position and angle of the implant based on the center point. Based on the derived center point, the implant placement path reflecting the anatomical characteristics is calculated, and the optimal alignment of the implant is designed through this. For example, it is possible to provide the ability to automatically adjust the position and orientation of the implant to meet a set inclination and version relative to the glenoid plane.

Additionally, the processor 102 may perform the function of designing and manufacturing a surgical guide in 3D. By creating a customized guide based on the center point and implant placement data and physically manufacturing it using technologies such as 3D printing, the accuracy of the surgery may be further improved. This guide may be used to guide a surgical instrument or alignment pin along a path optimized for the patient's anatomy.

Implant design may also be implemented through the processor 102. Based on the derived center point, a customized implant is automatically designed by considering the patient's anatomical characteristics and implant design requirements. The customized implant may be manufactured to precisely fit the patient's glenoid structure, maximizing the stability and success of the surgical outcomes.

As a result, the present invention may additionally implement technologies such as surgical guidance design, optimal implant placement, surgical guide production, and customized implant design, in addition to deriving the center point, through the processor 102. These features enable precise surgical planning tailored to the patient's anatomical characteristics and greatly contribute to improving the accuracy and efficiency of the surgical outcomes.

Additionally, the present invention includes a method for setting the center point through specification by the user in the process of determining the center point of the glenoid. This is to allow the user to review the data directly and adjust the center point to suit the characteristics of the surgical plan, in addition to the center point calculation method provided by the automated algorithm.

The user's specification approach is particularly useful when there is significant anatomical variation or specific requirements regarding implant placement. The processor provides the user with a 3D visualization of the glenoid and surrounding anatomical structures based on the CT or MRI data. The user may view the circumference of the glenoid and the generated intersections through a display screen, and review the inclination and version data along with the probability values for respective intersections.

The user may then select a specific intersection point through the provided interface or fine-tune the center point candidates produced by the existing automated algorithm. The specification by the user is performed via mouse clicks, touch input, or input devices, and the selected center point is saved as a reference point for surgical planning and guidance design.

In addition, the present invention intuitively displays data such as probability values, inclination, and versions of center point candidates to support the user's specification. The probability values may be visualized by color or shading to easily compare the likelihood of each intersection, and the inclination and version values may be displayed numerically to quantitatively evaluate anatomical aptness of the center point. This allows the user to freely adjust the position of the center point within a clinically reasonable range.

The user's specification approach may be used in parallel with the automated center point calculation method, allowing the user's expert judgment to be reflected in the final center point selection process. The present invention thereby combines the efficiency of the automated algorithm with the user's anatomical judgment, enabling more precise and patient-specific center point determination.

The above-described embodiments are merely examples, and other embodiments may be implemented within the scope of the appended claims.

### [Reference Numerals]

100: Electronic apparatus
101: Input/output interface
102: Processor
103: Memory

## Claims

1. A method of determining a center point in a patient's glenoid, performed by a processor of an electronic apparatus, the method comprising:
acquiring an image of the patient's glenoid;
obtaining a circumference of the glenoid;
defining a plurality of areas based on criteria set with respect to respective points of the circumference;
obtaining intersections formed by overlapping the plurality of defined areas and calculating a probability of each intersection; and
determining a center point based on a calculated probability value.

2. The method of claim 1, wherein the determining of the center point based on the calculated probability value comprises selecting the intersection at which the probability value is maximum as the center point.

3. The method of claim 2, wherein the determining of the center point based on the calculated probability value comprises, if the intersection point with the maximum probability value exceeds preset threshold values of inclination and version, selecting the intersection with the next probability rank as the center point.

4. The method of claim 1, wherein the obtaining of the circumference of the glenoid comprises:
extracting an edge of a glenoid surface by segmenting the image of the glenoid and analyzing an inclination change or density difference of the glenoid surface; and
obtaining the circumference of the glenoid along an extracted edge line of the glenoid surface.

5. The method of claim 1, wherein the defining of the plurality of areas based on the criteria set with respect to the respective points of the circumference comprises representing an area formed by a plurality of circles having a same radius centered at the respective points of the circumference.

6. The method of claim 5, wherein the defining of the plurality of areas based on the criteria set with respect to the respective points of the circumference comprises representing an area formed with a variable radius according to anatomical characteristics of the glenoid centered on the respective points of the circumference.

7. The method of claim 1, further comprising visually displaying the probability of each intersection by the processor, wherein the probability is displayed using a color, shading, or a numerical value.

8. The method of claim 7, further comprising displaying, by the processor, to a user inclination and version values of each intersection along with the probability value to allow the user to select a center point candidate.

9. An electronic apparatus for determining a center point in a patient's glenoid, the electronic apparatus comprising:
an input/output interface which receives an image of the glenoid;
a processor configured to:
obtain a circumference of the glenoid from the image of the glenoid;
define areas based on criteria set with respect to respective points on the circumference;
obtain intersections of the defined areas; and
determine a center point based on probability values of the obtained intersections; and
a memory configured to store or output data of the center point.

10. The electronic apparatus according to claim 9, wherein the processor is further configured to determine a final center point based on respective probability values of a plurality of intersections, and inclination values and version values at the plurality of intersections.
